# EUROPÄISCHE PATENTANMELDUNG

(11) **EP 4 190 393 A1**
(43) Veröffentlichungstag der Anmeldung: **07.06.2023**
(21) Anmeldenummer: 21212073.7
(22) Anmeldetag: 02.12.2021
(51) Int. Cl.: A61N 1/05, A61N 1/362, A61N 1/368

(54) **ELEKTRODE FÜR EIN KARDIALES STIMULATIONSSYSTEM**

(71) Anmelder: BIOTRONIK SE & Co. KG, 12359 Berlin (DE)
(72) Erfinder: Wenzel, Matthias, 10243 Berlin (DE); Jadwitzak, Detmar, 15537 Erkner (DE); Kaiser, Dajana, 12247 Berlin (DE); Fründt, Carsten, 13057 Berlin (DE); Hillebrand, Gordon, 12309 Berlin (DE); Schurr, Marc Steffen, 10179 Berlin (DE); TSEMO KAMGA, Joel Alain, 64367 Mühltal (DE); JOCHUM, Tobias, 13187 Berlin (DE)
(74) Vertreter: Biotronik Corporate Services SE

(57) **Zusammenfassung**

Eine Elektrode (11-13) für ein kardiales Stimulationssystem (1) umfasst ein distales Ende (110, 120, 130), ein proximales Ende (111, 121, 131), eine im Bereich des distalen Endes (110, 120, 130) angeordnete Elektrodenanordnung und einen an dem proximalen Ende (111, 121, 131) angeordneten Steckverbinder (112, 122, 132) zur Verbindung mit einem Pulsgenerator (10) des Stimulationssystems (1), wobei der Steckverbinder (112, 122, 132) eine Mehrzahl von elektrischen Kontakten (B, C1-C3) zur elektrischen Kontaktierung mit Gegenkontakten (E) des Pulsgenerators (10) aufweist. Dabei ist vorgesehen, dass die Elektrodenanordnung eine Mehrzahl von Paaren von Elektrodenpolen (125-127) aufweist, wobei ein jedes Paar von Elektrodenpolen (125-127) einen Anodenpol (A1-A6) und einen Kathodenpol (K1-K6) umfasst, wobei zumindest einige der Anodenpole (A1-A6) oder zumindest einige der Kathodenpole (K1-K6) gemeinsam an einen der Kontakte (B, C1-C3) des Steckverbinders (112, 122, 132) angeschlossen sind.

## Beschreibung

Die vorliegende Erfindung betrifft eine Elektrode für ein kardiales Stimulationssystem, insbesondere ein CRT-System für die kardiale Resynchronisationstherapie.

Eine solche Elektrode weist ein distales Ende, ein proximales Ende, eine im Bereich des distalen Endes angeordnete Elektrodenanordnung und einen an dem proximalen Ende angeordneten Steckverbinder zur Verbindung mit einem Pulsgenerator des Stimulationssystems auf. Der Steckverbinder umfasst eine Mehrzahl von elektrischen Kontakten, die zur elektrischen Kontaktierung mit Gegenkontakten des Pulsgenerators ausgebildet sind.

Ein Stimulationssystem beispielsweise in Form eines CRT-Systems dient zur sogenannten kardialen Resynchronisationstherapie (Englisch "Cardiac Resynchronisation Therapy", kurz CRT). Bei einigen Patienten mit einer chronischen Herzschwäche (so genannter Herzinsuffizienz) ist die natürliche Erregung zur Kontraktion der Herzkammern gestört. Bei einem sogenannten Linksschenkelblock kann hierbei insbesondere die Erregung der linken Herzkammer (linker Ventrikel) derart beeinträchtigt sein, dass die linke Herzkammer nur unzureichend und zudem nicht synchron mit der rechten Herzkammer kontrahiert. Eine Folge ist, dass die Herzkammern nicht mehr in einer aufeinander abgestimmten Weise arbeiten, das Herz somit unregelmäßig schlägt und dadurch die Pumpleistung des Herzens reduziert ist.

Ziel der kardialen Resynchronisationstherapie ist es somit, die Erregung der Herzkammern so zu beeinflussen, dass die Synchronität zwischen den Herzkammern wiederhergestellt wird, indem die rechte und linke Herzkammer durch eingespeiste elektrische Erregung zeitgleich stimuliert werden. Ein CRT-System verwendet zu diesem Zweck mehrere Elektroden, die gemeinsam mit einem Pulsgenerator implantiert und von denen eine in die rechte Herzkammer (rechter Ventrikel) und eine andere an oder auf die linke Herzkammer (linker Ventrikel) verlegt werden, Beispielsweise durch den Sinus coronarius. Eine dritte Elektrode kann zusätzlich im rechten Vorhof (rechtes Atrium) angeordnet sein, um eine Abstimmung mit einer Vorhofaktivität zu ermöglichen. Ein solches CRT-System kann mit einer reinen Herzschrittmacherfunktion ausgestaltet sein (sogenanntes CRT-P-System). Weil Patienten mit einer chronischen Herzschwäche häufig auch einem erhöhten Risiko eines plötzlichen Herzstillstands (plötzlicher Herztod) unterliegen, kann ein CRT-System zusätzlich aber auch eine Defibrillator-Funktion zur hochenergetischen Anregung zum Zwecke der Defibrillation aufweisen (sogenanntes CRT-D-System).

Wünschenswert bei einem Stimulationssystem, insbesondere einem CRT-System, ist die Möglichkeit für eine flexible, auf spezifische Symptome eines Patienten abgestimmte Stimulation. Zur Stimulation wird hierbei üblicherweise ein Strom, insbesondere in Form eines gepulsten Gleichstroms, über die Elektrodenanordnung der Elektrode abgegeben und in Gewebe injiziert, wobei der Strom so zu bemessen ist, dass eine Reizschwelle zur Anregung des Gewebes überschritten wird und somit eine Anregung einer Aktivität, insbesondere einer Herzaktivität, bewirkt werden kann.

Hierbei ist jedoch zu beachten, dass aufgrund der Feldverteilung zwischen Elektrodenpolen der Elektrodenanordnung es gegebenenfalls zu einer ungewünschten Stimulation von Bereichen kommen kann, die nicht angeregt werden sollen, zum Beispiel einer Stimulation des Nervus Phrenicus im Bereich des Herzens. Zudem kann, um eine Reizschwelle lokal zu überwinden, das Einspeisen eines vergleichsweise großen Stroms erforderlich sein, um an einem bestimmten lokalen Ort eine hinreichende Feldstärke zu erzielen.

Wünschenswert ist somit eine Elektrode, bei der eine Stimulation in effektiver Weise erfolgen kann, mit einer reduzierten Gefahr einer ungewünschten Stimulation von Nerven, zum Beispiel des Nervus Phrenicus im Bereich des Herzens.

Aus der US 7,792,585 B1 ist eine Elektrode bekannt, bei der Gruppen von Elektrodenpolen im Bereich eines distalen Endes der Elektrode vorgesehen sind.

Die US 2014/0005742 A1 beschreibt ein Verfahren, bei dem schnell und effizient kardiale Anregungsvektoren zur Minimierung einer Stimulation des Nervus Phrenicus bestimmt werden können.

Die US 2016/0346552 A1 beschreibt ein Stimulationssystem, bei dem ein Pulsgenerator eine Schalteinheit zum Schalten zwischen Elektrodenpolen aufweist, um einen Stimulationsvektor einzustellen.

Aufgabe der vorliegenden Erfindung ist es, eine Elektrode für ein kardiales Stimulationssystem zur Verfügung zu stellen, die eine Stimulation in effektiver Weise ermöglicht, bei einem reduzierten Risiko einer ungewünschten Stimulation von Nerven, zum Beispiel des Nervus Phrenicus im Bereich des Herzens.

Diese Aufgabe wird durch einen Gegenstand mit den Merkmalen des Anspruchs 1 gelöst.

Demnach weist die Elektrodenanordnung eine Mehrzahl von Paaren von Elektrodenpolen auf, wobei ein jedes Paar von Elektrodenpolen einen Anodenpol und einen Kathodenpol umfasst, wobei zumindest einige der Anodenpole oder zumindest einige der Kathodenpole gemeinsam an einen der Kontakte des Steckverbinders angeschlossen sind.

Elektroden sind über ihre Steckverbinder üblicherweise an zugeordnete Steckverbinderanschlüsse eines Anschlussblocks des Pulsgenerators anzuschließen. Die Steckverbinderanschlüsse des Pulsgenerators sind hierbei zum Beispiel vorbestimmten Elektroden, beispielsweise einer Elektrode zur linksventrikulären Stimulation, einer Elektrode zur rechtsventrikulären Stimulation und einer Elektrode zur Stimulation im rechten Atrium, zugeordnet. Über eine Steuereinrichtung des Pulsgenerators werden die Steckverbinderanschlüsse und darüber die angeschlossenen Elektroden angesteuert, um elektrische Stimulationspulse in bestimmungsgemäßer Weise abzugeben.

Die Einspeisung von Energie zur Gewebestimulation erfolgt bei einer Elektrode über die im Bereich des distalen Endes angeordnete Elektrodenanordnung, die durch eine Mehrzahl von Paaren von Elektrodenpolen gebildet ist. Ein jedes Paar umfasst hierbei einen Anodenpol und einen Kathodenpol, über die ein Strom in Gewebe injiziert und somit eine Stimulation bewirkt werden kann.

Dadurch, dass mehrere Paare von Elektrodenpolen vorgesehen sind, kann eine verteilte Anregung an Gewebe erfolgen. Dies ermöglicht zum einen eine lokale, begrenzte Stimulation, was das Überschreiten einer Reizschwelle lokal unter Verwendung einer vergleichsweise kleinen Stromstärke eines eingespeisten Stroms ermöglicht. Zudem kann eine Ausbreitung eines Anregungsfeldes lokal begrenzt sein, sodass eine ungewünschte Stimulation in weiter entfernt liegenden Bereichen, zum Beispiel an Nerven wie dem Nervus Phrenicus, vermieden werden kann.

Über die Paare von Elektrodenpolen ist insbesondere eine Modellierung eines Anregungsfeldes möglich, sodass eine Felddichte für eine Stimulation von Zellen lokal eingestellt werden kann. Eine solche Modellierung ermöglicht, eine am Stimulationssystem verwendete Spannung zu reduzieren, reduziert somit auch den Stromverbrauch und kann zu einer Lebensdauerverlängerung des Systems beitragen.

Der Steckverbinder der Elektrode kann beispielsweise vierpolig mit vier elektrischen Kontakten ausgestaltet sein. Entsprechend kann der Steckverbinder beispielsweise an einen vierpoligen Steckverbinderanschluss des Pulsgenerators angeschlossen werden.

Der Steckverbinder kann genormt sein und entsprechend an einen genormten Steckverbinderanschluss des Anschlussblocks anzuschließen sein. Beispielsweise kann es sich bei dem Steckverbinder um einen sogenannten IS4-Stecker, der nach der Norm ISO 27186:2010 (erste Ausgabe aus dem März 2010) genormt ist, oder einen DF4-Stecker handeln.

Dadurch, dass mehrere der Anodenpole oder mehrere der Kathodenpole an einen gemeinsamen Kontakt des Steckverbinders angeschlossen sind, kann die Anzahl der Kontakte des Steckverbinders von der Anzahl der Elektrodenpole der Elektrodenanordnung abweichen. Das gemeinsame Verbinden von einigen der Elektrodenpole mit einem zugeordneten, gemeinsamen Kontakt des Steckverbinders ermöglicht die Verwendung eines normierten Steckverbinders mit definierter Anzahl von Kontakten.

Die Elektrodenpole der Paare von Elektrodenpolen können insbesondere als Ringelektroden ausgestaltet sein, die sich ringförmig an einem Elektrodenkörper der Elektrode erstrecken. Die Elektrodenpole eines jeden Paares von Elektrodenpolen sind hierbei axial zueinander versetzt und somit axial zueinander beabstandet. Zudem sind auch die Paare von Elektrodenpolen paarweise axial zueinander versetzt und somit axial zueinander beabstandet.

In einer Ausgestaltung sind die zumindest einigen der Anodenpole oder die zumindest einigen der Kathodenpole über je eine gesonderte elektrische Leitung an den einen der Kontakte des Steckverbinders angeschlossen. Die Anodenpole oder die Kathodenpole sind somit über je eine gesonderte elektrische Leitung mit den Kontakten des Steckverbinders verbunden, wobei die Leitungen einiger der Anodenpole oder einiger der Kathodenpole elektrisch an einen gemeinsamen Kontakt des Steckverbinders angeschlossen und die Anodenpole bzw. die Kathodenpole somit elektrisch miteinander verbunden sind.

In alternativer Ausgestaltung können die zumindest einigen der Anodenpole oder die zumindest einigen der Kathodenpole über eine gemeinsame elektrische Leitung an den einen der Kontakte des Steckverbinders angeschlossen sein. Es erstreckt sich somit lediglich eine einzige Leitung zwischen dem gemeinsamen Kontakt und den zugeordneten Anodenpolen oder den zugeordneten Kathodenpolen. Auf diese Weise kann die Anzahl der erforderlichen elektrischen Leitungen, die in dem Elektrodenkörper der Elektrode aufzunehmen und zu verlegen sind, reduziert werden. Elektrisch sind wiederum die Anodenpole bzw. die Kathodenpole gemeinsam an einen zugeordneten Kontakt des Steckverbinders angeschlossen.

In einer Ausgestaltung sind zumindest einige der Anodenpole an einen ersten gemeinsamen Kontakt des Steckverbinders angeschlossen, und zumindest einige der Kathodenpole sind gemeinsam an einen zweiten gemeinsamen Kontakt des Steckverbinders angeschlossen. Sowohl einige der Anodenpole als auch einige der Kathodenpole sind damit jeweils an einen gemeinsamen Kontakt angeschlossen. Anodenpole und Kathodenpole sind damit jeweils elektrisch miteinander verschaltet und gruppenweise einem gemeinsamen Kontakt zugeordnet.

In einer Ausgestaltung ist ein Anodenpol eines Paares von Elektrodenpolen mit einem Kathodenpol eines Paares von Elektrodenpolen elektrisch verbunden. Es besteht somit eine elektrische Verbindung im Sinne eines Kurzschlusses zwischen zumindest einem der Anodenpole und zumindest einem der Kathodenpole der Paare von Elektrodenpolen. Insbesondere bei Verwendung einer Vielzahl von Paaren von Elektrodenpolen, insbesondere mehr als drei Paaren von Elektrodenpolen, kann eine solche Verschaltung für eine Einstellung einer gewünschten Feldverteilung sinnvoll sein.

Eine Verschaltung von Anodenpolen und/oder Kathodenpolen miteinander kann beispielsweise über eine oder mehrere elektrische Brückungsleitungen erfolgen, die zwischen einander zugeordneten Anodenpolen und/oder Kathodenpolen erstreckt sind und einen Kurzschluss zwischen den jeweils zugeordneten Anodenpolen und/oder Kathodenpolen herstellen. In diesem Fall kann eine einzige, gemeinsame elektrische Leitung zur Verbindung einer Gruppe von Anodenpolen und/oder Kathodenpolen mit einem zugeordneten Kontakt des Steckverbinders verwendet werden. Die Anodenpole und/oder Kathodenpole sind durch eine oder mehrere Brückungsleitungen zu einer gemeinsamen Gruppe zusammengefasst und über die zugeordnete Leitung mit dem Kontakt des Steckverbinders verbunden.

In einer Ausgestaltung weisen der Anodenpol und der Kathodenpol zumindest eines der Paare von Elektrodenpolen einen ersten Abstand zueinander auf. Dieser erste Abstand kann vergleichsweise klein sein und beispielsweise zwischen 1 mm und 5 mm, zum Beispiel 1,5 mm betragen. Der Abstand kann hierbei beispielsweise zwischen einer axialen Mitte des zum Beispiel als Ringelektrode ausgeführten Anodenpols und der axialen Mitte des zum Beispiel ebenfalls als Ringelektrode ausgeführten Kathodenpols gemessen sein.

Benachbarte Paare von Elektrodenpolen können demgegenüber deutlich weiter voneinander entfernt sein. So kann ein Paar von Elektrodenpolen zum Beispiel einen zweiten Abstand zu einem benachbarten Paar von Elektrodenpolen, vorzugsweise zu allen benachbarten Paaren von Elektrodenpolen, aufweisen, der deutlich größer als der erste Abstand ist. Beispielweise kann der zweite Abstand zwischen 10 mm und 30 mm, zum Beispiel 15 mm betragen, gemessen zum Beispiel zwischen den axialen Mitten einander benachbarter Elektrodenpole der unterschiedlichen Paare von Elektrodenpolen.

Dadurch, dass der Anodenpol und der Kathodenpol eines jeden Paares von Elektrodenpolen vergleichsweise dicht beieinander angeordnet ist, kann eine lokal begrenzte Feldverteilung im Bereich eines jeden Paares von Elektrodenpolen eingestellt werden, was eine Stimulation bei vergleichsweise geringer einzuspeisender Energie auf einen vergleichsweise kleinen Bereich begrenzt. Über die axial verteilten Paare von Elektrodenpolen kann hierbei eine verteilte, räumlich versetzte Anregung bewirkt werden, die für eine effiziente Gewebestimulation angepasst sein kann.

In einer Ausgestaltung sind die Anodenpole sämtlicher Paare von Elektrodenpolen an einen gemeinsamen Kontakt der Kontakte des Steckverbinders angeschlossen. Sämtliche Anodenpole sind somit einem einzigen Kontakt des Steckverbinders zugeordnet. Dieser Kontakt kann beispielsweise, in einer Ausgestaltung, proximal an dem Steckverbinder angeordnet und zum Beispiel durch einen Kontaktstift des Steckverbinders verwirklicht sein. Weitere Kontakte des Steckverbinders, die den Kathodenpolen der unterschiedlichen Paare von Elektrodenpolen zugeordnet sind, können demgegenüber distal zu dem den Anodenpolen zugeordneten Kontakt des Steckverbinders angeordnet sein und sind beispielsweise durch ringförmige Kontakte des Steckverbinders verwirklicht.

In einer Ausgestaltung weist die Elektrode eine Fixierungseinrichtung auf, die zur Fixierung der Elektrode im implantierten Zustand an Gewebe dient. Die Fixierungseinrichtung ist am distalen Ende der Elektrode angeordnet und kann beispielsweise durch eine helixförmige Schraube, eine Spule, einen oder mehrere Haken nach Art von Widerhaken, einen oder mehrere Befestigungsdrähte oder dergleichen verwirklicht sein und dient dazu, die Elektrode im Bereich des distalen Endes ortsfest zu umgebendem Gewebe zu fixieren. Die Elektrodenpaare der Elektrodenanordnung sind hierbei vorzugsweise proximal der Fixierungseinrichtung an der Elektrode angeordnet, indem die Fixierungseinrichtung distal zu dem am weitesten distal gelegenen Paar von Elektrodenpolen gelegen ist.

Ein Stimulationssystem weist vorzugsweise einen Pulsgenerator mit einem Anschlussblock auf, der einen oder mehrere Steckverbinderanschlüsse ausbildet. Eine oder mehrere Elektroden der beschriebenen Art können an einen oder mehrere der Steckverbinderanschlüsse des Anschlussblocks angeschlossen werden, um die Elektroden funktional mit dem Pulsgenerator zu verbinden. Über den Pulsgenerator können insbesondere Stimulationspulse erzeugt und in die Elektroden eingespeist werden, um eine Anregung an Gewebe zu bewirken.

Das Stimulationssystem kann insbesondere als CRT-System zur kardialen Resynchronisationstherapie ausgestaltet sein. Vorzugsweise können hierbei mehrere Elektroden zur Anregung an unterschiedlichen Orten im menschlichen Herzen, insbesondere im rechten Ventrikel und an oder auf dem linken Ventrikel und gegebenenfalls zusätzlich im rechten Vorhof, an den Pulsgenerator anzuschließen sein.

Der der Erfindung zugrunde liegende Gedanke soll nachfolgend anhand der in den Figuren dargestellten Ausführungsbeispiele näher erläutert werden. Es zeigen:
- Fig. 1: eine Ansicht des menschlichen Herzens mit einem implantierten Stimulationssystem;
- Fig. 2: eine schematische Ansicht eines Anschlussblocks eines Pulsgenerators des Stimulationssystems;
- Fig. 3: eine Ansicht eines Ausführungsbeispiels einer Elektrode mit einem Steckverbinder und einer im Bereich eines distalen Endes angeordneten Elektrodenanordnung;
- Fig. 4: eine ausschnittsweise vergrößerte Ansicht der Elektrode im Bereich des Steckverbinders und im Bereich der Elektrodenanordnung;
- Fig. 5: eine schematische Ansicht der elektrischen Verschaltung von Elektrodenpolen der Elektrodenanordnung mit Kontakten des Steckverbinders;
- Fig. 6: eine andere schematische Ansicht der elektrischen Verschaltung;
- Fig. 7: eine Ansicht der Elektrode im Bereich des menschlichen Herzens;
- Fig. 8: eine schematische Ansicht einer Anregung an Gewebe unter Verwendung der Elektrode;
- Fig. 9: eine Ansicht eines Ausführungsbeispiels einer Elektrodenanordnung an einer Elektrode;
- Fig. 10: eine Ansicht eines anderen Ausführungsbeispiels einer Elektrodenanordnung an einer Elektrode; und
- Fig. 11: eine Ansicht eines wiederum anderen Ausführungsbeispiels einer Elektrodenanordnung an einer Elektrode.

Im folgenden werden Ausführungsbeispiele mit Bezug auf die Figuren beschrieben. In den Figuren sind hierbei Bauteile gleicher Funktion mit gleichen Bezugszeichen versehen.

Bereits an dieser Stelle sei angemerkt, dass die beschriebenen Ausführungsbeispiele nicht als beschränkend für die vorliegende Erfindung zu verstehen sind, sondern lediglich der Illustration dienen.

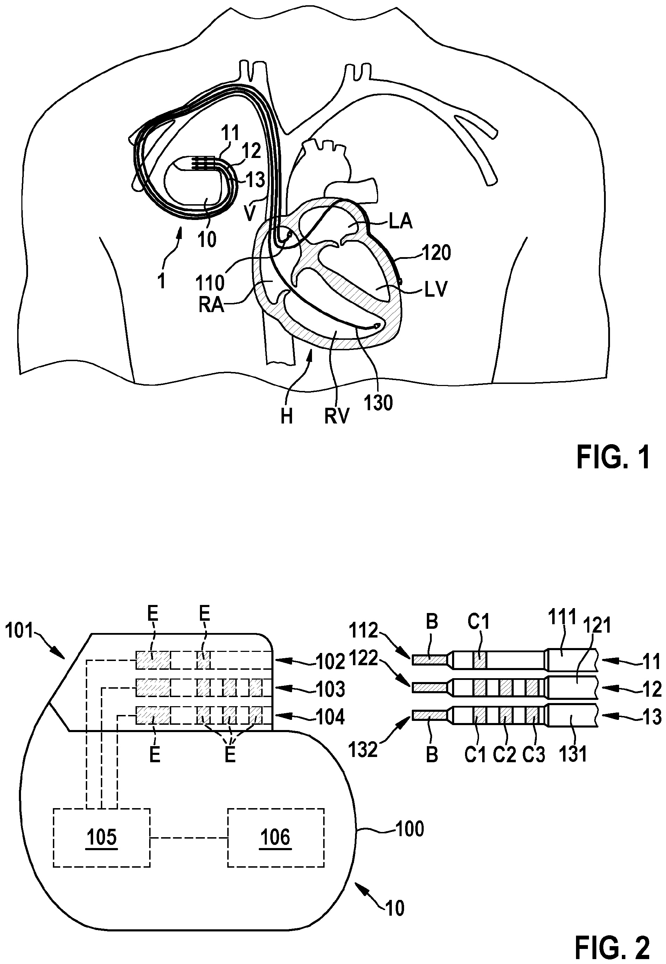

Fig. 1 zeigt in einer schematischen Ansicht das Herz H eines Patienten mit einem implantierten Stimulationssystem 1 zum Beispiel in Form eines CRT-Systems. Das Stimulationssystem 1 weist einen Pulsgenerator 10 auf, an den Elektroden 11, 12, 13 angeschlossen sind. Der Pulsgenerator 10 ist zusammen mit den Elektroden 11, 12, 13 im Patienten implantiert derart, dass sich die Elektroden 11, 12, 13 ausgehend vom Pulsgenerator 10 durch eine obere Hohlvene V hin zum Herzen H erstrecken und zu unterschiedlichen Stimulationsorten im Herzen H verlegt sind.

Bei dem dargestellten Beispiel sind drei Elektroden 11, 12, 13 an den Pulsgenerator 10 angeschlossen. Der Pulsgenerator 10 ist beispielsweise subkutan im Bereich des Schlüsselbeins des Patienten implantiert. Von dem Pulsgenerator 10 sind die Elektroden 11, 12, 13 derart verlegt, dass die Elektroden 11, 12, 13 mit ihren distalen Enden 110, 120, 130 im rechten Atrium RA (Elektrode 11 mit dem distalen Ende 110), außen am linken Ventrikel LV (Elektrode 12 mit dem distalen Ende 120) und im rechten Ventrikel RV (Elektrode 13 mit dem distalen Ende 130) zu liegen kommen und somit über durch den Pulsgenerator 10 generierte und in die Elektroden 11, 12, 13 eingeleitete Stimulationspulse eine Stimulation an unterschiedlichen Stimulationsorten im Herzen H erfolgen kann.

Im Rahmen einer kardialen Resynchronisationstherapie erfolgt eine Stimulation am linken Ventrikel LV und im rechten Ventrikel RV, sodass durch die Stimulation eine Synchronität der Ventrikelaktivität bewirkt werden kann. Auf diese Weise soll bei Patienten mit einer chronischen Herzschwäche (Herzinsuffizienz) die Pumpleistung des Herzens H erhöht werden.

Wie schematisch in einem Ausführungsbeispiel in Fig. 2 dargestellt ist, weist der Pulsgenerator 10 ein Gehäuse 100 auf, in dem elektrische und elektronische Komponenten in Form einer Steuereinrichtung 105 und einer Energieversorgungseinrichtung 106 in Form einer Batterie eingefasst und gekapselt sind.

An dem Gehäuse 100 ist ein Anschlussblock 101 angeordnet, der Steckverbinderanschlüsse 102, 103, 104 in Form von Buchsenverbindern aufweist, in die Elektroden 11, 12, 13 mit zugeordneten Steckverbindern 112, 122, 132 in Form von Steckern eingesteckt werden können, um die Elektroden 11, 12, 13 und die Steckverbinderanschlüsse 102, 103, 104 und somit den Pulsgenerator 10 elektrisch miteinander zu kontaktieren.

Die Steckverbinderanschlüsse 102, 103, 104 sind vorzugsweise genormt und sind beispielsweise als IS-1- oder IS4-Anschlüsse nach ISO 27186:2010 ausgestaltet. Die Steckverbinderanschlüsse 102, 103, 104 weisen jeweils eine Anzahl von elektrischen Kontaktelementen E auf, wobei ein erster Steckverbinderanschluss 102 beispielsweise zwei elektrische Kontaktelemente E und die anderen beiden Steckverbinderanschlüsse 103, 104 jeweils vier elektrische Kontaktelemente E aufweisen können. Der Steckverbinderanschluss 102 ist somit als zweipoliger Anschluss ausgestaltet, während die anderen Steckverbinderanschlüsse 103, 104 als vierpolige Anschlüsse ausgebildet sind.

Entsprechend der Ausgestaltung der Steckverbinderanschlüsse 102, 103, 104 sind die Steckverbinder 112, 122, 132 der Elektroden 11, 12, 13 genormt und beispielsweise als IS-1-Stecker (Steckverbinder 112) oder IS4-Stecker (Steckverbinder 122, 132) ausgestaltet und weisen zwei elektrische Kontaktelemente B, C1 (zweipoliger Steckverbinder 112) oder vier elektrische Kontaktelemente B, C1-C3 (vierpolige Steckverbinder 122, 132) auf.

Die Kontaktelemente E auf Seiten der Steckverbinderanschlüsse 102, 103, 104 und die Kontaktelemente B, C1-C3 auf Seiten der Steckverbinder 112, 122, 132 sind derart ausgestaltet, dass beim steckenden Verbinden eine paarweise elektrische Kontaktierung zwischen den jeweils zugeordneten Kontaktelementen B, C1-C3, E hergestellt wird.

Im Rahmen eines CRT-Systems sind die Steckverbinderanschlüsse 102, 103, 104 üblicherweise jeweils bestimmten Elektroden 11, 12, 13 zugeordnet. So dient ein Steckverbinderanschluss 102 zum Anschließen einer Elektrode 11, die in das rechte Atrium RA zu verlegen ist. Ein zweiter Steckverbinderanschluss 103 dient demgegenüber zum Anschließen einer linksventrikulären Elektrode 12. Ein Steckverbinderanschluss 104 dient zum Anschließen einer rechtsventrikulären Elektrode 13.

Bei dem in Fig. 1 dargestellten Pulsgenerator 10 werden über die Steuereinrichtung 105 elektrische Stimulationspulse generiert und über die Steckverbinderanschlüsse 102, 103, 104 an die zugeordneten Elektroden 11, 12, 13 abgegeben. Bei einem vierpoligen Steckverbinderanschluss 103, 104 für eine linksventrikuläre Elektrode 12 oder eine rechtsventrikuläre Elektrode 13 können hierbei in mehrpoliger Weise unterschiedliche zeitversetzte Stimulationspulse generiert und abgegeben werden, wobei eine zeitliche Verzögerung zwischen zum Beispiel den linksventrikulären Stimulationspulsen einstellbar ist.

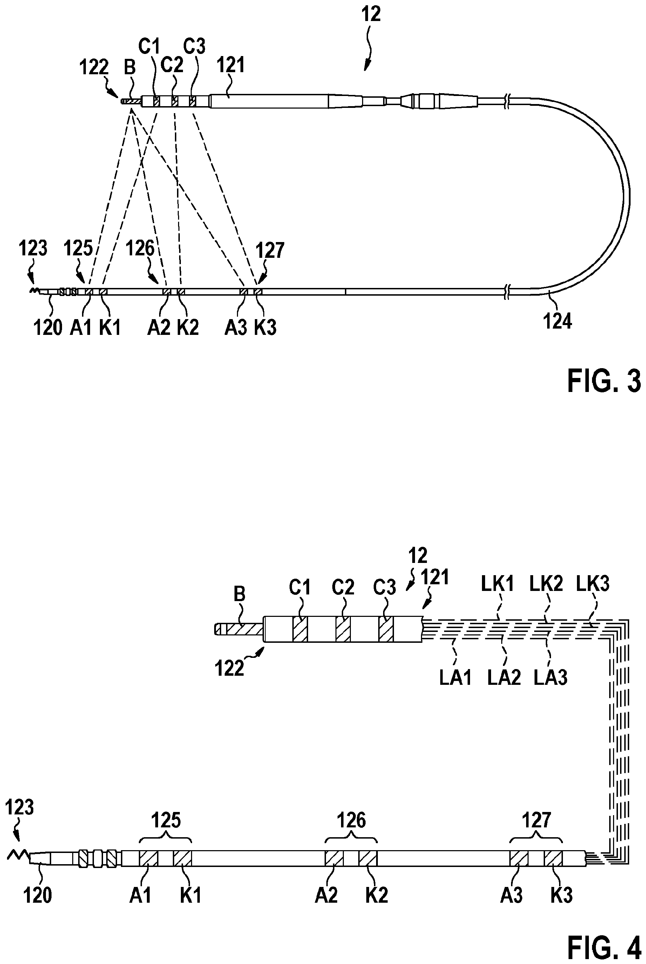

Fig. 3 zeigt ein Ausführungsbeispiel einer Elektrode 12, die einen Elektrodenkörper 124 mit einem distalen Ende 120 und einem proximalen Ende 121 aufweist. Im Bereich des distalen Endes 120 ist eine Elektrodenanordnung bestehend aus Paaren von Elektrodenpolen 125, 126, 127 angeordnet. Im Bereich des proximalen Endes 121 ist demgegenüber ein Steckverbinder 122 angeordnet, der zum elektrischen Verbinden mit einem zugeordneten Steckverbinderanschluss 102-104 eines Pulsgenerators 10 dient.

Wie aus Fig. 3 in Zusammenschau mit der vergrößerten Ansicht gemäß Fig. 4 ersichtlich, weist ein jedes Elektrodenpolpaar 125-127 einen Anodenpol A1-A3 und einen Kathodenpol K1-K3 auf. Der Anodenpol A1-A3 und der Kathodenpol K1-K3 eines jeden Paares von Elektrodenpolen 125-127 sind vergleichsweise dicht beieinander angeordnet, wobei die Paare von Elektrodenpolen 125-127 axial entlang der Längserstreckungsrichtung des Elektrodenkörpers 124 zueinander beabstandet und - im Vergleich des Abstands der Pole eines einzelnen Paares - vergleichsweise weit voneinander entfernt sind.

Wie schematisch in Fig. 4 in Zusammenschau mit Fig. 3 dargestellt, sind bei dem dargestellten Ausführungsbeispiel die Anodenpole A1-A3 über elektrische Leitungen LA1-LA3 mit einem zugeordneten, gemeinsamen Kontakt B des Steckverbinders 122 verbunden, sodass die Anodenpole A1-A3 gemeinsam an einen einzigen Kontakt B angeschlossen sind. Der Kontakt B ist proximal am Steckverbinder 122 angeordnet und durch einen Kontaktstift geformt.

Die Kathodenpole K1-K3 sind demgegenüber jeweils durch eine gesonderte Leitung LK1-LK3 mit einem jeweils zugeordneten Kontakt C1-C3 des Steckverbinders 122 verbunden, sodass die Kathodenpole K1-K3 bei dem dargestellten Ausführungsbeispiel an gesonderte Kontakte C1-C3 des Steckverbinders 122 angeschlossen sind.

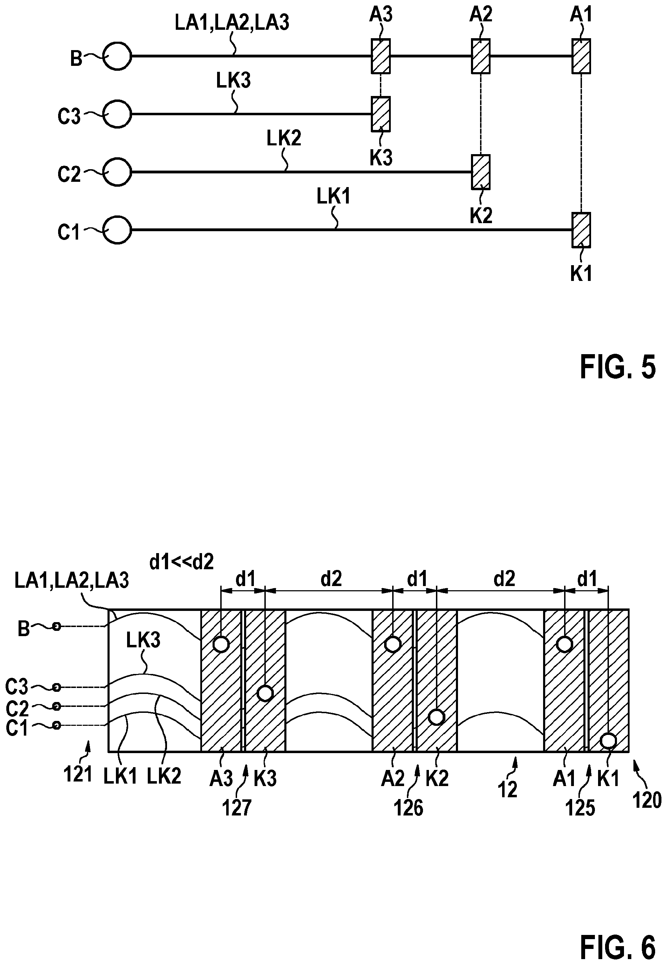

Dies ist schematisch auch in Fig. 5 dargestellt. Während die Anodenpole A1-A3 an einen gemeinsamen Kontakt B angeschlossen sind, sind die Kathodenpole K1-K3 mit gesonderten Kontakten C1-C3 des Steckverbinders 122 verbunden.

Der Anschluss der Anodenpole A1-A3 an den gemeinsamen Kontakt B kann über gesonderte elektrische Leitungen LA1-LA3 erfolgen, wie dies schematisch in Fig. 4 dargestellt ist, sodass für einen jeden Anodenpol A1-A3 eine gesonderte Leitung im Elektrodenkörper 124 verlegt und hin zu dem Kontakt B erstreckt ist. Die gesonderten Leitungen LA1-LA3 sind hierbei gemeinsam an dem Kontakt B festgelegt und somit gemeinsam an den Kontakt B elektrisch angeschlossen.

Alternativ kann, wie dies schematisch in Fig. 6 dargestellt ist, eine gemeinsame Leitung LA1, LA2, LA3 verwendet werden, die an sämtliche Anodenpole A1-A3 angeschlossen ist, sodass die Anodenpole A1-A3 über eine einzige, gemeinsame Leitung an den zugeordneten Kontakt B angeschlossen sind.

Wie zudem aus Fig. 6 ersichtlich ist, sind der Anodenpol A1-A3 und der Kathodenpol K1-K3 eines jeden Paares von Elektrodenpolen 125-127 um einen Abstand d1 zueinander beabstandet, gemessen zum Beispiel zwischen der axialen Mitte der einander zugeordneten Pole. Der Abstand d1 zwischen den Polen eines jeden Paares 125-127 ist hierbei klein gegenüber dem Abstand d2 benachbarter Paare zueinander. Der Abstand d2 kann hierbei gemessen werden zum Beispiel zwischen den axialen Mitten einander zugewandter Pole der benachbarten Paare.

Beispielsweise kann der Abstand d1 zwischen den Polen eines Paares in einem Bereich zwischen 1 mm und 5 mm, beispielsweise bei 1,5 mm liegen. Der Abstand d2 zwischen benachbarten Paaren kann demgegenüber zum Beispiel in einem Bereich zwischen 10 mm und 30 mm, zum Beispiel bei 15 mm liegen.

Die Anodenpole A1-A3 und die Kathodenpole K1-K3 können zum Beispiel jeweils als Ringelektroden ausgebildet sein und erstrecken sich somit ringförmig im Bereich des distalen Endes 120 am Elektrodenkörper 124.

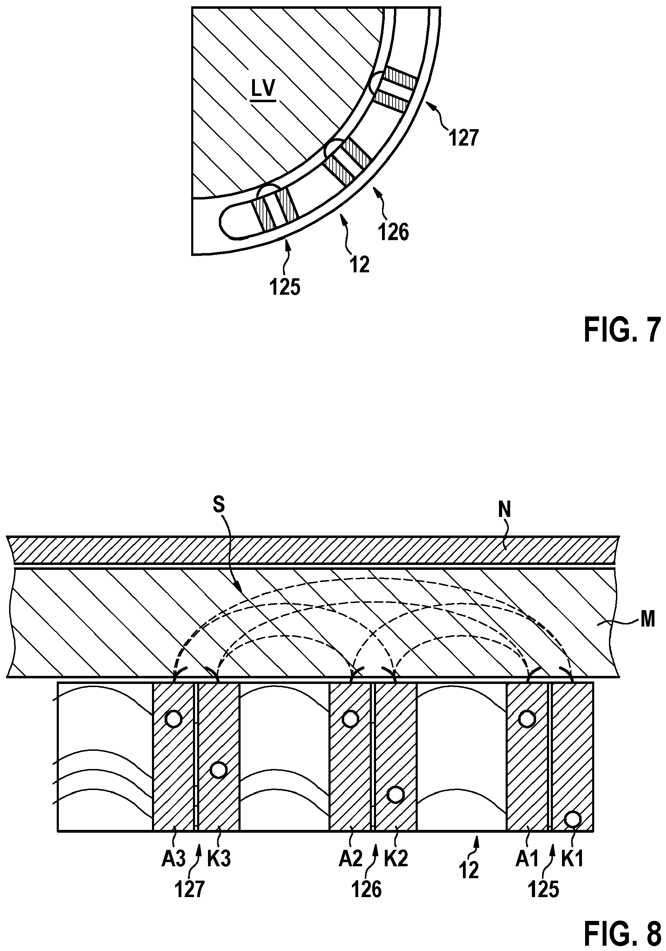

Durch Verwendung von Paaren von Elektrodenpolen 125-127, gebildet jeweils durch einen Anodenpol A1-A3 und einen Kathodenpol K1-K3, wird eine Elektrodenanordnung geschaffen, bei der eine räumlich verteilte, örtlich begrenzte Anregung zur Gewebestimulation bereitgestellt werden kann. Beispielsweise kann eine solche Elektrode 12, wie dies in Fig. 7 dargestellt ist, in den Bereich eines koronaren Sinus implantiert sein, um eine Stimulation im Bereich eines Ventrikels, zum Beispiel des linken Ventrikel LV, zu bewirken. Durch Injektion von Strompulsen über die Paare von Elektrodenpolen 125-127 kann eine Feldverteilung S, wie in Fig. 8 dargestellt, erzeugt werden, die eine vergleichsweise starke Anregung mit örtlich großer Stromdichte unmittelbar im Bereich der Paare von Elektrodenpolen 125-127 bewirkt, jedoch in das Gewebe M hinein vergleichsweise schnell abfällt und sich somit insbesondere nicht (in einer für eine Anregung wirksamen Weise) hin zu umgebenden Nerven N, insbesondere dem Nervus Phrenicus erstreckt.

Durch die Anordnung der Paare von Elektrodenpolen 125-127 und zudem durch geeignete Ansteuerung kann eine örtlich und auch zeitlich gewünschte Anregung im Sinne einer modellierten Feldverteilung eingestellt werden, die eine effiziente Gewebestimulation bei vergleichsweise kleiner Stromstärke und kleiner anzulegender Spannung ermöglicht.

Über die Paare von Elektrodenpolen 125-127 kann insbesondere eine monofokale, bifokale oder trifokale Stimulation bewirkt werden. Bei einer monofokalen Stimulation erfolgt eine Anregung lediglich über eines der Elektrodenpolpaare 125-127. Bei der bifokalen Stimulation erfolgt demgegenüber eine Stimulation über zwei der Elektrodenpolpaare 125-127. Und bei einer trifokalen Stimulation erfolgt eine Stimulation über sämtliche Elektrodenpolpaare 125-127. Bei der bifokalen Stimulation oder trifokalen Stimulation kann ein Einspeisen von Erregungspulsen gleichzeitig oder mit einem zeitlichen Versatz erfolgen, wobei die Erregungspulse gleich oder unterschiedlich geformt sein können und auch eine gleiche oder unterschiedliche Energie aufweisen können.

Wie dies aus Fig. 3 und 4 ersichtlich ist, ist am distalen Ende 120 der Elektrode 12 eine Fixierungseinrichtung 123 angeordnet, die zur Fixierung der Elektrode 12 in implantiertem Zustand an Gewebe dient. Die Fixierungseinrichtung 123 kann beispielsweise durch eine helixförmige Schraube, durch Fixierungsdrähte, durch eine Spule, durch Haken oder auf andere Weise verwirklicht sein und wird bei Implantation mit umgebendem Gewebe in Wirkverbindung gebracht, sodass die Elektrode 12 im Bereich des distalen Endes 120 über die Fixierungseinrichtung 123 zu dem Gewebe festgelegt wird.

Bei den Ausführungsbeispielen gemäß Fig. 3 bis 6 sind drei Paare von Elektrodenpolen 125-127 vorgesehen. Denkbar und möglich ist aber auch, weniger oder mehr Paare von Elektrodenpolen 125-127 zu verwenden, beispielsweise zwei Paare oder mehr als drei, zum Beispiel 4, 5, 6, 7, 8 oder sogar mehr Paare von Elektrodenpolen.

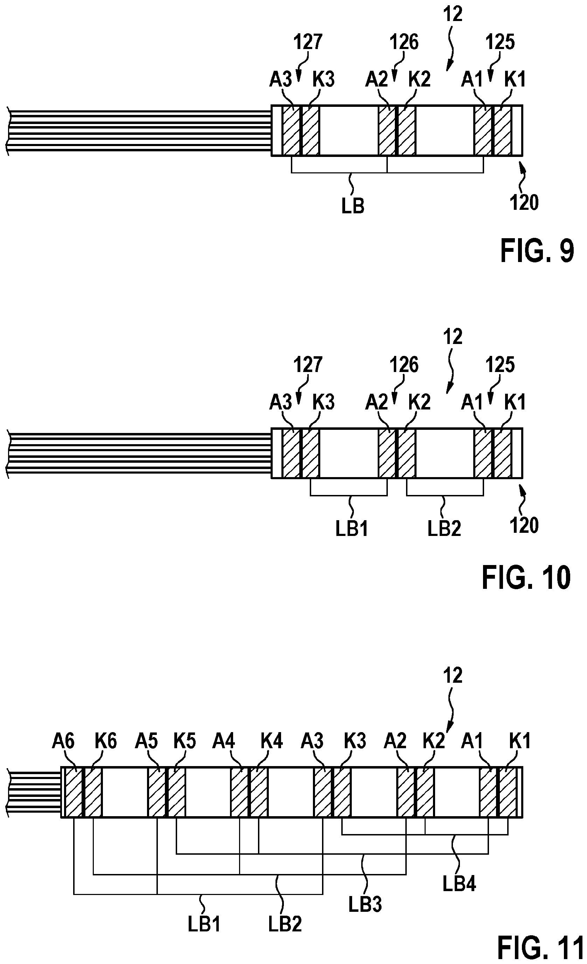

In einem in Fig. 9 dargestellten Ausführungsbeispiel sind drei Paare von Elektrodenpolen 125-127 vorgesehen, die im Bereich des distalen Endes 120 der Elektrode 12 angeordnet sind. Entsprechend den Ausführungsbeispielen gemäß Fig. 3 bis 6 sind die Anodenpole A1-A3 der Paare 125-127 elektrisch miteinander verbunden, wobei dies bei dem dargestellten Ausführungsbeispiel über eine Brückungsleitung LB erfolgt, die die Anodenpole A1-A3 miteinander kurzschließt.

Im Vergleich zum Ausführungsbeispiel gemäß Fig. 9 sind bei dem Ausführungsbeispiel gemäß Fig. 10 Anodenpole A1-A3 und Kathodenpole K1-K3 elektrisch miteinander über Brückungsleitungen LB1, LB2 kurzgeschlossen. So ist der Anodenpol A1 des Paares 125 mit dem Kathodenpol K2 des benachbarten Paares 126 verbunden. Der Anodenpol A2 des Paares 126 ist wiederum elektrisch mit dem Kathodenpol K3 des Paares 127 verbunden.

Bei einem in Fig. 11 dargestellten Ausführungsbeispiel sind insgesamt sechs Paare von Elektrodenpolen vorgesehen, die jeweils einen Anodenpol A1-A6 und einen Kathodenpol K1-K6 aufweisen. Über Brückungsleitungen LB1-LB4 sind einige der Anodenpole A1-A6 (die Anodenpole A3, A4, A6 über die Brückungsleitung LB1), einige der Kathodenpole K1-K6 (die Kathodenpole K1-K3 über die Brückungsleitung LB4) und einige der Anodenpole A1-A6 und Kathodenpole K1-K6 kreuzweise (der Anodenpol A1 und die Kathodenpole K4, K5 über die Brückungsleitung LB3 und die Anodenpole A2, A3 und der Kathodenpol K6 über die Brückungsleitung LB2) miteinander kurzgeschlossen.

Der der Erfindung zugrunde liegende Gedanke ist nicht auf die vorangehend beschriebenen Ausführungsbeispiele beschränkt, sondern lässt sich auch in anderer Weise verwirklichen.

Insbesondere können auch andere Verteilungen von Elektrodenpolen denkbar möglich sein. Zum Beispiel kann bei einer Elektrodenanordnung eine andere Anzahl von Paaren von Elektrodenpolen mit jeweils einer Anode und einer Kathode als bei dem dargestellten Ausführungsbeispielen vorgesehen sein.

### Liste der Bezugszeichen

- 1: Stimulationssystem (CRT-System)
- 10: Pulsgenerator
- 100: Gehäuse
- 101: Anschlussblock
- 102-104: Steckverbinderanschluss
- 105: Steuereinrichtung
- 106: Energieversorgungseinrichtung
- 11: Elektrode
- 110: Distales Ende
- 111: Proximales Ende
- 112: Steckverbinder
- 12: Elektrode
- 120: Distales Ende
- 121: Proximales Ende
- 122: Steckverbinder
- 123: Fixierungseinrichtung
- 124: Elektrodenkörper
- 125-127: Paar von Elektrodenpolen
- 13: Elektrode
- 130: Distales Ende
- 131: Proximales Ende
- 132: Steckverbinder
- A1-A6: Anodenpol
- B, C1, C2, C3: Kontakt
- d1, d2: Abstand
- E: Gegenkontakt
- H: Herz
- K1-K6: Kathodenpol
- LA: Linkes Atrium
- LA1-LA3, LK1-LK3: Elektrische Leitung
- LB, LB1-LB4: Brückungsleitung
- LV: Linkes Ventrikel
- M: Herzgewebe (Myokard)
- N: Nervus Phrenicus
- RA: Rechtes Atrium
- RV: Rechtes Ventrikel
- S: Feldverteilung
- V: Obere Hohlvene

## Patentansprüche

1. Elektrode (11-13) für ein kardiales Stimulationssystem (1), mit einem distalen Ende (110, 120, 130), einem proximalen Ende (111, 121, 131), einer im Bereich des distalen Endes (110, 120, 130) angeordneten Elektrodenanordnung und einem an dem proximalen Ende (111, 121, 131) angeordneten Steckverbinder (112, 122, 132) zur Verbindung mit einem Pulsgenerator (10) des Stimulationssystems (1), wobei der Steckverbinder (112, 122, 132) eine Mehrzahl von elektrischen Kontakten (B, C1-C3) zur elektrischen Kontaktierung mit Gegenkontakten (E) des Pulsgenerators (10) aufweist, **dadurch gekennzeichnet, dass** die Elektrodenanordnung eine Mehrzahl von Paaren von Elektrodenpolen (125-127) aufweist, wobei ein jedes Paar von Elektrodenpolen (125-127) einen Anodenpol (A1-A6) und einen Kathodenpol (K1-K6) umfasst, wobei zumindest einige der Anodenpole (A1-A6) oder zumindest einige der Kathodenpole (K1-K6) gemeinsam an einen der Kontakte (B, C1-C3) des Steckverbinders (112, 122, 132) angeschlossen sind.

2. Elektrode (11-13) nach Anspruch 1, **dadurch gekennzeichnet, dass** der Steckverbinder (112, 122, 132) ein IS4-Anschluss ist.

3. Elektrode (11-13) nach Anspruch 1 oder 2, **dadurch gekennzeichnet, dass** die zumindest einigen der Anodenpole (A1-A6) oder die zumindest einigen der Kathodenpole (K1-K6) über je eine gesonderte elektrische Leitung (LA1-LA3, LK1-LK3) an den einen der Kontakte (B, C1-C3) des Steckverbinders (112, 122, 132) angeschlossen sind.

4. Elektrode (11-13) nach Anspruch 1 oder 2, **dadurch gekennzeichnet, dass** die zumindest einigen der Anodenpole (A1-A6) oder die zumindest einigen der Kathodenpole (K1-K6) über eine gemeinsame elektrische Leitung (LA1-LA3, LK1-LK3) an den einen der Kontakte (B, C1-C3) des Steckverbinders (112, 122, 132) angeschlossen sind.

5. Elektrode (11-13) nach einem der vorangehenden Ansprüche, **dadurch gekennzeichnet, dass** zumindest einige der Anodenpole (A1-A6) an einen ersten gemeinsamen Kontakt (B, C1-C3) des Steckverbinders (112, 122, 132) angeschlossen sind und zumindest einige der Kathodenpole (K1-K6) gemeinsam an einen zweiten gemeinsamen Kontakt (B, C1-C3) des Steckverbinders (112, 122, 132) angeschlossen sind.

6. Elektrode (11-13) nach einem der vorangehenden Ansprüche, **dadurch gekennzeichnet, dass** ein Anodenpol (A1-A6) eines Paares von Elektrodenpolen (125-127) mit einem Kathodenpol (K1-K6) eines anderen Paares von Elektrodenpolen (125-127) elektrisch verbunden ist.

7. Elektrode (11-13) nach einem der vorangehenden Ansprüche, **dadurch gekennzeichnet, dass** zumindest einige der Anodenpole (A1-A6) und/oder zumindest einige der Kathodenpole (K1-K6) über eine elektrische Brückungsleitung (LB, LB1-LB3) miteinander verbunden sind.

8. Elektrode (11-13) nach einem der vorangehenden Ansprüche, **dadurch gekennzeichnet, dass** der Anodenpol (A1-A6) und der Kathodenpol (K1-K6) zumindest eines der Paare von Elektrodenpolen (125-127) einen ersten Abstand (d1) zueinander aufweisen, wobei das zumindest eine der Paare von Elektrodenpolen (125-127) in einem zweiten Abstand (d2), der größer als der erste Abstand (d1) ist, zu einem benachbarten Paar von Elektrodenpolen (125-127) angeordnet ist.

9. Elektrode (11-13) nach Anspruch 8, **dadurch gekennzeichnet, dass** der erste Abstand (d1) in einem Bereich zwischen 1 mm und 5 mm und der zweite Abstand (d2) in einem Bereich zwischen 10 mm und 30 mm liegt.

10. Elektrode (11-13) nach einem der vorangehenden Ansprüche, **dadurch gekennzeichnet, dass** die Anodenpole (A1-A6) sämtlicher Paare von Elektrodenpolen (125-127) an einen gemeinsamen Kontakt (B) der Kontakte (B, C1-C3) des Steckverbinders (112, 122, 132) angeschlossen sind.

11. Elektrode (11-13) nach Anspruch 10, **dadurch gekennzeichnet, dass** der gemeinsame Kontakt (B) proximal an dem Steckverbinder (112, 122, 132) angeordnet ist und weitere Kontakte (C1-C3) der Kontakte (B, C1-C3) des Steckverbinders (112, 122, 132), die mit den Kathodenpolen (K1-K6) der Paare von Elektrodenpolen (125-127) elektrisch verbunden sind, distal relativ zu dem den Anodenpolen (A1-A6) zugeordneten gemeinsamen Kontakt (B) angeordnet sind.

12. Elektrode (11-13) nach einem der vorangehenden Ansprüche, **gekennzeichnet durch** eine am distalen Ende (120) der Elektrode (11, 12, 13) angeordnete Fixierungseinrichtung (123) zur Fixierung der Elektrode an Gewebe (M).

13. Elektrode (11-13) nach Anspruch 12, **dadurch gekennzeichnet, dass** die Fixierungseinrichtung (123) distal relativ zu einem dem distalen Ende (110, 120, 130) der Elektrode (11, 12, 13) am weitesten angenäherten Paar der Paare von Elektrodenpolen (125-127) angeordnet ist.

14. Stimulationssystem (1), mit einem Pulsgenerator (10), der einen Anschlussblock (101) mit zumindest einem Steckverbinderanschluss (102-104) aufweist, und zumindest einer an den zumindest einen Steckverbinderanschluss (102-104) anschließbaren Elektrode (11, 12, 13) nach einem der vorangehenden Ansprüche.

15. Stimulationssystem (1) nach Anspruch 14, **dadurch gekennzeichnet, dass** das Stimulationssystem (1) ein CRT-System (1) zur kardialen Resynchronisationstherapie ist.
